# EUROPÄISCHE PATENTSCHRIFT

(11) **EP 0 611 752 B1**
(45) Veröffentlichungstag und Bekanntmachung des Hinweises auf die Patenterteilung: **04.09.1996**
(21) Anmeldenummer: 94101810.3
(22) Anmeldetag: 07.02.1994
(51) Int. Cl.: C07C 269/02, C07C 271/20, C07C 271/24, C08G 18/80, C08G 18/28

(54) **Urethangruppen enthaltende, olefinisch ungesättigte Isocyanate und ihre Verwendung als Bindemittel in Beschichtungsmaterialien**
Urethane groups containing olefinically unsaturated isocyanates and their use as binders in coating compositions
Isocyanates oléfiniquement insaturés ayant de groupement uréthane utilisables comme liants dans les compositions de revêtement

(30) Priorität: 19.02.1993 DE 4305162
(43) Veröffentlichungstag der Anmeldung: 24.08.1994
(73) Patentinhaber: BAYER AG, 51368 Leverkusen (DE)
(72) Erfinder: Brahm, Martin, Dr., D-51766 Engelskirchen (DE); Arning, Eberhard, Dipl.-Ing., D-41564 Kaarst (DE); Schmalstieg, Lutz, Dr., D-50676 Köln (DE); Riberi, Bernd, Dr., D-51519 Odenthal (DE)

(56) Entgegenhaltungen:
- EP-A- 0 301 345
- EP-A- 0 451 657
- DE-A- 1 495 983
- DE-A- 2 726 900
- FR-A- 2 084 434

## Beschreibung

Die Erfindung betrifft ein Verfahren zur Herstellung von olefinisch ungesättigten Isocyanaten durch Umsetzung von ausgewählten, vorzugsweise cycloaliphatischen Polyisocyanaten mit ausgewählten olefinisch ungesättigten, vorzugsweise einwertigen Alkoholen, die nach diesem Verfahren erhältlichen Isocyanate und ihre Verwendung als Bindemittel in Beschichtungsmaterialien.

Einkomponentig zu verarbeitende Beschichtungsmittel auf Basis NCO-funktioneller Urethangruppen enthaltender Prepolymere sind seit langem bekannt (Houben Weyl, Methoden der Organischen Chemie, Band E 20, Seite 1646, Georg Thieme Verlag 1987). Sie werden durch Umsetzung von organischen Polyisocyanaten mit höherfunktionellen Polyolen, insbesondere Polyether- und/oder Polyesterpolyolen hergestellt. Der höhermolekulare Aufbau verleiht diesen Verbindungen gute filmbildende und filmoptische Eigenschaften, jedoch ebenso eine höhere Viskosität, weshalb sie nur stark verdünnt oder mit erheblichem Anteil an monomeren Diisocyanat als Lackbindemittel bzw. als Beschichtungsmaterial eingesetzt werden können. Hohe Konzentrationen an monomerem Diisocyanat sind jedoch aus physiologischer Sicht nicht akzeptabel. Darüber hinaus ist der Einsatz von großen Mengen an Lösungsmittel umweltpolitisch nicht vertretbar. Die Schere zwischen einem hochmolekularen, hochfunktionellen Aufbau mit hoher Viskosität aber positiven Produkteigenschaften einerseits und niedermolekularen niedrigviskosen Produkten aber ungenügender Lösungsbeständigkeit und ungenügenden Trocknungseigenschaften andererseits läßt sich durch NCO-funktionelle Prepolymere nach dem oben erwähnten Aufbauprinzip nicht schließen.

Neben NCO-funktionellen Beschichtungsmaterialien sind auch oxidativ vernetzbare 1 K-Systeme beschrieben (Ullmann, Enzyklopädie der technischen Chemie, 4. Auflage, Band 19, Seite 75 ff, Verlag Chemie Weinheim, Deerfield Beach Florida, Basel 1980). Diese polymeren Verbindungen können zusätzlich auch Urethangruppen enthalten (DE-OS 4 011 376). Weisen diese oxidativ vernetzenden Urethanharze einen höhermolekularen Aufbau auf, so ergeben sich relativ schnell trocknende Lacksysteme, mit guter Beständigkeit. Jedoch sind auch für derartige Materialien zur Verarbeitung erhebliche Mengen an Lösungsmittel bzw. reaktivem Verdünner erforderlich.

Reaktive Verdünner wie beispielsweise beschrieben in EP-A-0 301 345 weisen zwar lösungsmittelfrei relativ niedrige Viskositäten auf, können jedoch nur in Verbindung mit erheblichen Mengen an hochmolekularen Verbindungen wie Alkydharzen zu Beschichtungsmaterialien verarbeitet werden. Als Alleinbindemittel haben sie keine bzw. ungenügende filmbindende Eigenschaften und bedingt durch den niedermolekularen Aufbau eine ungenügende und zu langsame Trocknung.

Estergruppenenthaltende Reaktivverdünner, wie beispielsweise in EP-A-0 301 345 beschrieben, sind für Anwendungen im Baubereich ungeeignet, da auf basischen Untergründen wie Beton die Gefahr einer schnellen Spaltung der hydrolytisch anfälligen Estergruppe besteht.

Es war daher die der Erfindung zugrundeliegende Aufgabe, neue, niedrigviskose Bindemittel für lösungsmittelfreie bzw. -arme Beschichtungsmaterialien mit guten lacktechnischen Eigenschaften, schneller chemischer Trocknung bei Raumtemperatur und universeller und physiologisch unbedenklicher Anwendbarkeit zur Verfügung zu stellen.

Diese Aufgabe konnte mit dem nachstehend näher beschriebenen erfindungsgemäßen Verfahren gelöst werden, welches zu Verfahrensprodukten führt, die den genannten Anforderungen wegen des gleichzeitigen Vorliegens von olefinischen Doppelbindungen, Urethangruppen und Isocyanatgruppen genügen. Wesentlich ist hierbei insbesondere ein ausgewogenes Verhältnis von Urethangruppen, Isocyanatgruppen und zur oxidativen Vernetzung befähigten Doppelbindungen.

Gegenstand der Erfindung ist ein Verfahren zur Herstellung von olefinisch ungesättigten, Urethangruppen aufweisenden Isocyanaten mit einem NCO-Gehalt von 4 bis 20 Gew.-% und einer (mittleren) NCO-Funktionalität von 0,6 bis 1,5, dadurch gekennzeichnet, daß man
a) eine Isocyanatkomponente mit einem NCO-Gehalt von 20 bis 56 Gew.-% und einer (mittleren) NCO-Funktionalität von unter 2,5, bestehend im wesentlichen aus
   a1) 80 bis 100 Gew.-% an Urethangruppen-freien, cycloaliphatischen Diisocyanaten mit einem NCO-Gehalt von 25 bis 56 Gew.-% und
   a2) 0 bis 20 Gew.-% an anderen organischen Polyisocyanaten mit einem NCO-Gehalt von 10 bis 50 Gew.-%
   mit
b) einer olefinisch ungesättigten Alkoholkomponente einer (mittleren) Hydroxylfunktionalität von unter 1,5 und einer Iodzahl von über 70, bestehend im wesentlichen aus
   b1) 80 bis 100 Gew.-% einwertigen, olefinisch ungesättigten Alkoholen oder Gemischen derartiger Alkohole mit (im Mittel) 10 bis 22 Kohlenstoffatomen pro Molekül und
   b2) 0 bis 20 Gew.-% anderen ein- oder mehrwertigen Alkoholen des Molekulargewichts Bereich 32 bis 326,
gegebenenfalls in Anwesenheit von Katalysatoren für die NCO/OH-Additionsreaktion unter Einhaltung eines NCO/OH-Äquivalentverhältnisses von 4 bis 40 umsetzt und anschließend die Umsetzungsprodukte destillativ von überschüssigen, destillierbaren Ausgangsisocyanaten a1) und/oder a2) bis zu einem Restgehalt an solchen Isocyanaten von maximal 0,5 % befreit.

Gegenstand sind auch die nach diesem Verfahren erhältlichen, olefinisch ungesättigten Isocyanate.

Gegenstand der Erfindung ist schließlich auch die Verwendung dieser Isocyanate als Bindemittel für bei Raumtemperatur einkomponentig zu verarbeitende Beschichtungsmaterialien.

In der EP-A 0 301 345 sind zwar bereits Isocyanatgruppen aufweisende Umsetzungsprodukte von organischen Diisocyanaten mit ungesättigten Alkoholen beschrieben, jedoch handelt es sich hierbei um Isocyanatgruppen aufweisende Umsetzungsprodukte von kurzkettigen Alkoholen, die ohne Entfernung von nicht umgesetzten Ausgangsdiisocyanaten mit Polyolen vollständig unter Esterbildung umgesetzt werden. Nach diesem Verfahren hergestellte, im wesentlichen Isocyanatgruppen-freie, olefinisch ungesättigte Verbindungen weisen ungenügende Trocknungseigenschaften auf und neigen teilweise zur Kristallisation.

Die erfindungsgemäßen Verfahrensprodukte weisen die bereits oben genannten Kenndaten auf. Vorzugsweise liegt ihr NCO-Gehalt bei 6 bis 13 Gew.-%, ihre (mittlere) NCO-Funktionalität bei 0,7 bis 1,3, ihre Iodzahl bei 20 bis 250, ihre Viskosität bei 23°C bei 100 bis 5000 mPa·s und ihr Gehalt an destillierbaren Ausgangsisocyanaten unter 0,2 Gew.-%.

Beim erfindungsgemäßen Verfahren werden als Isocyanatkomponente a) cycloaliphatische Diisocyanate a1) oder Gemische von cycloaliphatischen Diisocyanaten a1) mit anderen organischen Polyisocyanaten a2) eingesetzt, wobei diese, falls überhaupt, in Mengen von bis zu 20, vorzugsweise bis zu 10 Gew.-%, bezogen auf das Gesamtgewicht der Komponente a) zum Einsatz gelangen.

Unter "cycloaliphatischen Diisocyanaten" sind solche Diisocyanate zu verstehen, die ausschließlich an aliphatische oder cycloaliphatische Kohlenstoffatome gebundene NCO-Gruppen und außerdem mindestens einen cycloaliphatischen Ring aufweisen. Die cycloaliphatischen Diisocyanate a1) weisen einen NCO-Gehalt von 25 bis 56 Gew.-%, vorzugsweise 30 bis 50 Gew.-% auf. Beispielhaft genannt seien 1,3-Diisocyanato-cyclopentan, 1,3- und 1,4-Diisocyanato-cyclohexan, 1-Methyl-2,4-diisocyanato-cyclohexan, 1-Methyl-2,6-diisocyanato-cyclohexan, die isomeren Diisocyanatodicyclohexylmethane, 2,5- und 2,6-Bis(isocyanatomethyl)dicyclo[2,2,1]heptan, 4-Isocyanato-methyl-1-methyl-cyclohexylisocyanat (IMCI), 1-Isocyanato-3,3,5-trimethyl-5-isocyanatomethyl-cyclohexan (IPDI), sowie 1,3-oder 1,4-Bis(dimethyl-isocyanatomethyl)cyclohexan. Auch beliebige Gemische derartiger Isocyanate können als Komponente a1) eingesetzt werden. Besonders bevorzugt wird als Komponente a1) IPDI verwendet.

Weitere Ausgangsisocyanate a2), die zur Erzielung spezieller Eigenschaften in untergeordneten Mengen mitverwendet werden können, sind solche mit einem NCO-Gehalt von 10 bis 50 Gew.-%. In Betracht kommen beipsielsweise aliphatische Diisocyanate wie 1,6-Diisocyanatohexan (HDI) oder 2,2,4-Triethyl-1,6-diisocyanatohexan oder auch aromatische Diisocyanate wie 2,4- und/oder 2,6-Diisocyanatotoluol oder die isomeren Diphenylmethandiisocyanate. Vorzugsweise handelt es sich bei den anderen Polyisocyanaten a2) jedoch um Modifizierungsprodukte von Polyisocyanaten der oben beispielhaft genannten Art mit Biuret-, Uretdion- und/oder Isocyanuratgruppen, die beispielsweise in situ durch eine Biuretisierung, Dimerisierung und/oder Trimerisierung eines Teils der Isocyanatgruppen von Diisocyanaten der als Komponente a1) bzw. b2) vorgesehenen Art erhalten werden. Diese Modifizierungsreaktionen können zu einem beliebigen Zeitpunkt abgebrochen werden, so daß Gemische a) aus den Einzelkomponenten a1) und a2) in den genannten Mengenverhältnissen resultieren.

Besonders bevorzugt werden ausschließlich cycloaliphatische Diisocyanate, insbesondere ausschließlich IPDI als Komponente a) eingesetzt.

Die Komponente b) weist vorzugsweise eine Hydroxylfunktionalität von 1 bis 1,5, vorzugsweise von 1, eine Iodzahl von über 90, vorzugsweise von 100 bis 400 auf.

Es handelt sich bei der Komponente b) um olefinisch ungesättigte Alkohole b1) oder um deren Gemische bis zu 20 Gew.-%, vorzugsweise bis zu 10 Gew.-% mit anderen Alkoholen b2). Vorzugsweise besteht die Komponente b) ausschließlich aus ungesättigten Alkoholen b1).

Geeignete Alkohole b1) sind olefinisch ungesättigte Alkohole oder Gemische von olefinisch ungesättigten Alkoholen mit (im Mittel) 10 bis 22, vorzugsweise 14 bis 20 Kohlenstoffatomen pro Molekül und Iodzahlen von über 70, vorzugsweise von über 90 und besonders bevorzugt von 100 bis 400. Gut geeignet sind beispielsweise einwertige Alkohole bzw. Alkoholgemische, die sich von den entsprechenden ungesättigten synthetischen oder natürlichen Fettsäuren bzw. Fettsäuregemischen ableiten. Beispielhaft genannt seien die durch Reduktion von ungesättigten Carbonsäuren wie Dodecensäure, Tetradecensäure, Hexadecensäure, Octadecensäure, Eicosensäure, Docosensäure, 12-Oxyoctadecensäure, Octadecadiensäure, Octadecatriensäure, Eicosatetraensäure, Docosapentaensäure, Decensäure oder Gemischen derartiger Säuren zu den entsprechenden Alkoholen erhalten werden.

Bei den gegebenenfalls mitverwendeten anderen Alkoholen b2) handelt es sich um solche des Molekulargewichtsbereich 32 bis 326, vorzugsweise 74 bis 286. Es kommen sowohl gesättigte einwertige als auch mehrwertige Alkohole in Betracht. Beispielhaft genannt seien Methanol, Ethanol, n-Propanol, Isopropanol, die isomeren Butanole, Pentanole oder Hexanole, n-Octanol, n-Dodecanol oder n-Octadecanol, gesättigte Fettalkohole oder mehrwertige Alkohole wie Ethylenglykol, Propylenglykol, die isomeren Butandiole, Hexandiole oder Octandiole, Glyzerin, Trimethylolpropan oder Gemische derartiger Alkohole. Die Alkohole b2) werden, falls überhaupt, in solchen Mengen innerhalb der genannten Bereiche eingesetzt, daß die genannte Bedingung bezüglich der Funktionalität der Komponente b) erfüllt ist.

Zur Durchführung des erfindungsgemäßen Verfahrens werden die Komponenten a) und b) in solchen Mengen miteinander zur Reaktion gebracht, die einem NCO/OH-Äquivalentverhältnis von 4:1 bis 40:1, vorzugsweise 5:1 bis 20:1 und besonders bevorzugt von 6:1 bis 12:1 entsprechen. Die Umsetzung erfolgt im allgemeinen innerhalb des Temperaturbereiches von 10 bis 140°C, vorzugsweise von 40 bis 100°C, wobei zur Beschleunigung gewünschtenfalls übliche Katalysatoren wie beispielsweise Triethylamin, Triethylendiamin, Dimethylbenzylamin, Zinn(II)octoat, Zinn(II) ethylhexanoat oder Dibutylzinkdilaurat mitverwendet werden können.

Im Anschluß an die Umsetzung wird daß überschüssige, destillierbare Ausgangsdiisocyanat destillativ, vorzugsweise durch Dünnschichtdestillation bis zu einem Restgehalt im Verfahrensprodukt von unter 0,5, vorzugsweise von unter 0,2 Gew.-% entfernt.

Bei den erfindungsgemäßen Verfahrensprodukten handelt es sich um wertvolle, unter dem Einfluß von Luftfeuchtigkeit und Luftsauerstoff aushärtbare Bindemittel für Beschichtungsmaterialien.

Vor der erfindungsgemäßen Verwendung der erfindungsgemäßen Verfahrensprodukte werden diesen im allgemeinen an sich bekannte, die oxidative Vernetzung beschleunigende Katalysatoren zugesetzt. Derartige Katalysatoren sind beispielsweise in Ullmann, Enzyklopädie der technischen Chemie, 4. Auflage, Band 23, Seite 42 (Trockenstoffe) Verlag Chemie 1983, sowie in der Offenlegungsschrift DE 4 032 546 und darin zitierten Schriften aufgeführt. Als Beispiele seien Kobalt-, Blei-, Magnesium-, Zirkonium-, Aluminium-, Mangan-, Calcium-, Cer-, Kupfer-, Nickel-, Vanadium-, Barium- und Zinksikkative sowie Gemische genannt.

Auch Katalysatoren zur Beschleunigung der Isocyanat-Additionsreaktion der oben beispielhaft genannten Art können den erfindungsgemäßen Verfahrensprodukten vor ihrer erfindungsgemäßen Verwendung zugesetzt werden.

Zur Erzeugung eines speziellen Eigenschaftsprofils der Beschichtungsmassen kann es sinnvoll sein, andere nichtfunktionelle Polymere bzw. NCO-funktionelle Zusätze und Polymerkomponenten, die zur oxidativen Vernetzung befähigt sind, als weitere Bindemittelkomponenten mitzuverwenden. Derartige weitere Bindemittelkomponenten werden im allgemeinen in einer Menge von maximal 30, vorzugsweise maximal 10 Gew.-% mitverwendet. Ganz besonders bevorzugt wird auf die Mitverwendung weiterer Bindemittelkomponenten bei der erfindungsgemäßen Verwendung der erfindungsgemäßen Verfahrensprodukte verzichtet.

Als weitere Bindemittelkomponenten kommen beispielsweise Alkydharze in Betracht wie sie z.B. in Römpps Chemielexikon, Band 1, Seite 202, Frankh'sche Verlagsbuchhandlung, Stuttgart, 1966 definiert oder bei D.H. Solomon, The Chemistry of Organic Filmformers, S. 75 bis 101, John Wiley & Sons Inc., New York 1967, beschrieben sind. NCO-funktionelle Bindemittelkomponenten, die neben den erfindungsgemäßen Verfahrensprodukten eingesetzt werden können sind beispielsweise die bekannten Lackpolyisocyanate, d.h. vorzugsweise (i) Urethangruppen, (ii) Isocyanurat- und/oder Uretdiongruppen oder (iii) Biuretgruppen aufweisende Derivate von aliphatischen Diisocyanaten, insbesondere von 1,6-Diisocyanatohexan.

Neben den erfindungsgemäßen Verfahrensprodukten und den beispielhaft genannten, gegebenenfalls mitverwendeten weiteren Bindemittelkomponenten können in den Beschichtungsmitteln auch andere Hilfs- und Zusatzmittel mitverwendet werden. Hierzu gehören beispielsweise Lösungsmittel, die allerdings nur in geringen Mengen mitverwendet werden, so daß der Feststoffanteil der Beschichtungsmaterialien über 85, vorzugsweise über 90 Gew.-% liegt, vorzugsweise wird auf die Mitverwendung von Lösungsmitteln verzichtet. Geeignete Lösungsmittel sind beispielsweise Toluol, Xylol, Cyclohexan, Chlorbenzol, Butylacetat, Ethylacetat, Ethylglykolacetat, Pentylacetat, Hexylacetat, Methoxypropylacetat, Tetrahydrofuran, Dioxan, Aceton, Methylethylketon, Xylol, Testbenzin, höhere Aromaten, wie sie beispielsweise unter der Bezeichnung ®Solvesso oder ®Shellsol im Handel erhältich sind, oder beliebige Gemische derartiger Lösungsmittel. Besonders gut geeignet sind geruchsarme isoparaffinische Lösungsmittel, wie sie beispielsweise unter den Bezeichnungen ®Isopar oder ®Nappar im Handel erhältich sind. Die erfindungsgemäßen Verfahrensprodukte weisen trotz der polaren Urethangruppen eine gute Verträglichkeit und Mischbarkeit mit diesen unpolaren Lösungsmitteln auf.

Als weitere Hilfsstoffe können in den erfindungsgemäßen Beschichtungsmaterialien auch beispielsweise die üblichen Benetzungsmittel, Verlaufsmittel, Hautverhinderungsmittel, Antischaummittel, Mattierungsmittel wie beispielsweise Kieselsäure, Aluminiumsilikate und hochsiedende Wachse, viskositätsregulierende Stoffe, Pigmente, Farbstoffe, UV-Absorber oder Stabilisatoren gegen thermischen bzw. oxidativen Abbau eingesetzt werden.

Die der erfindungsgemäßen Verfahrensprodukte als wesentliche Bindemittel enthaltende Beschichtungsmaterialien können zur Beschichtung beliebiger Substrate wie beispielsweise Holz, Kunststoff, Leder, Papier, Textilien, Glas, Keramik, Putz, Mauerwerk, Metalle oder Beton verwendet werden. Sie lassen sich mit üblichen Applikationsmethoden wie Spritzen, Streichen, Fluten, Gießen, Tauchen, Walzen aufbringen. Die Beschichtungsmittel können in Form von Klarlacken als auch in Form pigmentierter Lacke verwendet werden.

Die so hergestellten Beschichtungen härten bei 20°C im allgemeinen während eines Zeitraums von 2 bis 24 Stunden zu hochwertigen Überzügen aus. Die Härtung kann jedoch auch bei tieferen Temperaturen (bis -5°C) oder beschleunigt bei höheren Temperaturen (bis beispielsweise 130°C) erfolgen.

In den nachfolgenden Beispielen beziehen sich alle Angaben in "Teilen" und in "%" auf das Gewicht.

### Beispiele 1 bis 5 (erfindungsgemäß)

In einer mit Stickstoff gespülten Rührapparatur werden jeweils die in nachstehender Tabelle 1 angegebenen Mengen Isocyanat mit der ebenfalls angegebenen Menge Alkohol zur Reaktion gebracht, wozu die Isocyanatkomponente vorgelegt und bei 80°C mit der entwässerten Alkoholkomponente tropfenweise versetzt wird. Nach 3 bis 5 Stunden Reaktion unter Stickstoffatmosphäre ist der theoretische NCO-Gehalt erreicht. Nachfolgend wird das überschüssige Diisocyanat falls erforderlich durch Dünnschichtdestillation im Hochvakuum (0,1 bis 0,3 mbar) bei einer Temperatur von 150°C abgetrennt. In Tabelle 1 sind die Produktdaten zusammengefaßt.

**Tabelle 1**

| Beisp. Nr. | Isocyanat Menge in Val | Alkohol Menge in Val 18°C | NCO % | Viskosität (23°C) mPa·s | freies Diisocyanat % |
|---|---|---|---|---|---|
| 1¹⁾ | 10 IPDI | 1 ®Ocenol 110/130²⁾ | 8,48 | 700 | 0,10 |
| 2 | 7 IPDI | 1 ®Ocenol 110/130 | 8,14 | 800 | 0,04 |
| 3 | 10 IPDI | 1 ®Ocenol 110/130 0,3 TMP | 8,8 | 2100 | 0,49 |
| 4 | 10 Diisocyanatodicyclohexylmethan | 1 ®Ocenol 110/130 | 7,54 | 1500 | 0,03 |
| 5 | 8 IPDI trimerisiert³⁾ | 1 ®Ocenol 110/130 | 9,37 | 3500 | 0,05 |

| | | | | | |
|---|---|---|---|---|---|
| ¹⁾ Laut gelpermeationschromatographischer Analyse enthält das Produkt 85 % NCO-funktionelle Monourethanverbindung. | | | | | |
| ²⁾ Bei ®Ocenol 110/130 handelt es sich um ein technisches Gemisch einwertiger, olefinisch ungesättigter Alkohole der Fa. Henkel mit (im Mittel) 18 Kohlenstoffatomen pro Molekül und einer Iodzahl im Bereich von 110 bis 130. | | | | | |
| ³⁾ Das Diisocyanat wurde mit 0,2 mmol Methylcholin-Lösung (10%ig) von einem Start-NCO-Gehalt von 37,8 % auf einen NCO-Gehalt von 34,5 % bei 55°C antrimerisiert und mit 0,2 mmol Dibutylphosphat abgestoppt (theoretische Funktionalität: 2,07). Es handelt sich um ein Gemisch von ca. 82 Gew.-% IPDI mit ca. 18 Gew.-% IPDI-Trimerisat. | | | | | |

### Beispiele 6 bis 12 (Vergleichsbeispiele)

In der nachstehenden Tabelle 2 werden in Analogie zu Beispielen 1 bis 5 hergestellte Vergleichsprodukte beschrieben, die, wie aus Tabelle 2 ersichtlich, eine für die erfindungsgemäße Verwendung ungeeignete Konsistenz aufweisen, oder die, wie aus Tabelle 4 ersichtlich, unzureichende Trocknungseigenschaften aufweisen.

**Tabelle 2**

| Beisp. Nr. | Isocyanat Menge in Val | Alkohol Menge in Val | NCO % | Viskosität (23°C) mPa·s | freies Diisocyanat % |
|---|---|---|---|---|---|
| 6¹⁾ | 1 IPDI | 1 ®Ocenol 110/130 | <0,5 | 3300 | - |
| 7 | 2 IPDI | 1 ®Ocenol 110/130 | 6,3 | 1000 | 0,04 |
| 8 | 10 IPDI | 1 ®Ocenol 60/65²⁾ | 8,41 | 650 | 0,08 |
| 9 | 8 IPDI | 1 2-Ethyl-1,3-diol | 11,5 | harzartig | 0,03 |
| 10¹⁾ | 1 Diisocyanatodicyclohexylmethan | 1 ®Ocenol 110/130 | <0,5 | pastös | - |
| 11¹⁾ | 1 1,6-Hexamethylendiisocyanat | 1 ®Ocenol 110/130 | - | fest | - |
| 12¹⁾ | 1 2,4-Toluylendiisocyanat | 0,5 ®Ocenol 110/130 | 7,5 | kristallin | - |

| | | | | | |
|---|---|---|---|---|---|
| ¹⁾ nicht gedünnschichtetes Produkt | | | | | |
| ²⁾ Technisches Gemisch einwertiger, olefinisch ungesättigter Alkohole mit einer Iodzahl von 60 bis 65 und 12 bis 20 Kohlenstoffatome pro Molekül, Hersteller: Fa. Henkel, Düsseldorf | | | | | |

### Beispiele 13 bis 16 (erfindungsgemäße Verwendung)

Unter Verwendung der in nachstehender Tabelle 3 genannten erfindungsgemäßen Bindemittel werden Klarlacke der Zusammensetzung
- 93,4: Teile Lackbindemittel
- 2,8: Teile Octa Soligen Calcium 4
- 0,5: Teile Octa Soligen Kobalt 6
- 2,8: Teile Octa Soligen Zirkon 18
- 0,5: Teile Methylethylketoxim (Hautverhinderungsmittel)
hergestellt und auf gereinigte Glasplatten in einer Schichtdicke von 120 µm aufgetragen und bei Raumtemperatur ausgehärtet. Die resultierenden Trocknungszeiten (Sandtrocknung nach Stunden bei 20°C), Druckfestigkeiten (nach DIN 53150), Werte für die Pendeldämpfung (nach DIN 53157) und die Lösungsmittelfestigkeit (0 = unverändert, 5 = aufgelöst) werden in nachstehender Tabelle 3 zusammengestellt.

**Tabelle 3**

| Nr. | Bindemittel aus Beispiel | Sandtrocknung nach Stunden | druckfest nach Stunden | Pendeldämpfung nach 7 d in s | Lösungsmittelfestigkeit* |
|---|---|---|---|---|---|
| 13 | 1 | 4,5 | > 8 < 18 | 70 | 1 - 2 |
| 14 | 2 | 4,5 | > 8 < 18 | 55 | 1 - 2 |
| 15 | 3 | 4,0 | > 8 < 18 | 60 | 1 - 2 |
| 16 | 5 | 3,5 | > 8 < 18 | 60 | 1 |

### Beispiele 17 bis 19 (Vergleich)

Analog Beispielen 13 bis 16 wurden in den Beispielen 17 bis 19 Vergleichsformulierungen unter Verwendung der Produkte der Vergleichsbeispiele 6 bis 8 hergestellt. Die schlechten Trocknungseigenschaften gehen den aus den Daten der nachstehenden Tabelle 4 hervor.

**Tabelle 4**

| Nr. | Bindemittel aus Vergleichsbeisp. | Sandtrocknung nach Stunden | druckfest nach Stunden | Pendeldämpfung nach 7 d in s |
|---|---|---|---|---|
| 17 | 6 | > 24 | > 72 | 8 |
| 18 | 7 | > 24 | > 72 | 11 |
| 19 | 8 | > 24 | > 48 | 17 |

### Beispiel 19 (erfindungsgemäßer pigmentierter Lack)

Unter Verwendung des Bindemittels gemäß Beispiel 1 wird eine Basisformulierung für pigmentierte Lacke folgender Zusammensetzung hergestellt:
- 74,0: Teile Bindemittel 1
- 2,2: Teile Octa Soligen Calcium 4
- 18,6: Teile Bayertitan R-KB-5
- 2,2: Teile Toluolsulfonylisocyanat
- 0,4: Teile Octa Soligen Kobalt 6
- 2,2: Teile Octa Soligen Zirkon 18
- 0,4: Teile Ethlymethylketoxim

Ein auf eine gereinigte Glasplatte aufgetragener Film der Schichtdicke 120 µm ist bei 23°C nach 4 Stunden sandtrocken und nach 18 Stunden durchgetrocknet. Es ergibt sich eine lösungsmittelbeständige hochglänzende Lackoberfläche.

## Patentansprüche

1. Verfahren zur Herstellung von olefinisch ungesättigten, Urethangruppen aufweisenden Isocyanaten mit einem NCO-Gehalt von 4 bis 20 Gew.-% und einer (mittleren) NCO-Funktionalität von 0,6 bis 1,5, dadurch gekennzeichnet, daß man
a) eine Isocyanatkomponente mit einem NCO-Gehalt von 20 bis 56 Gew.-% und einer (mittleren) NCO-Funktionalität von unter 2,5, bestehend im wesentlichen aus
a1) 80 bis 100 Gew.-% an Urethangruppenfreien, cycloaliphatischen Diisocyanaten mit einem NCO-Gehalt von 25 bis 56 Gew.-% und
a2) 0 bis 20 Gew.-% an anderen organischen Polyisocyanaten mit einem NCO-Gehalt von 10 bis 50 Gew.-%
mit
b) einer olefinisch ungesättigten Alkoholkomponente einer (mittleren) Hydroxylfunktionalität von unter 1,5 und einer Iodzahl von über 70, bestehend im wesentlichen aus
b1) 80 bis 100 Gew.-% einwertigen, olefinisch ungesättigten Alkoholen oder Gemischen derartiger Alkohole mit (im Mittel) 10 bis 22 Kohlenstoffatomen pro Molekül und
b2) 0 bis 20 Gew.-% anderen ein- oder mehrwertigen Alkoholen des Molekulargewichts Bereich 32 bis 326,
gegebenenfalls in Anwesenheit von Katalysatoren für die NCO/OH-Additionsreaktion unter Einhaltung eines NCO/OH-Äquivalentverhältnisses von 4 bis 40 umsetzt und anschließend die Umsetzungsprodukte destillativ von überschüssigen, destillierbaren Ausgangsisocyanaten a1) und/oder a2) bis zu einem Restgehalt an solchen Isocyanaten von maximal 0,5 % befreit.

2. Verfahren gemäß Anspruch 1, dadurch gekennzeichnet, daß man als Komponente a) ausschließlich cycloaliphatische Diisocyanate a1) verwendet.

3. Verfahren gemäß Anspruch 1 und 2, dadurch gekennzeichnet, daß man als Komponente a) ausschließlich 1-Isocyanato-3,3,5-trimethyl-5-isocyanatomethylcyclohexan verwendet.

4. Verfahren gemäß Anspruch 1 bis 3, dadurch gekennzeichnet, daß man als Komponente b) ausschließlich Fettalkohole oder Gemische von Fettalkoholen mit einer Iodzahl von über 90 mit (im Mittel) 14 bis 20 Kohlenstoffatomen pro Molekül verwendet.

5. Gemäß Anspruch 1 bis 4 erhältliche, olefinisch ungesättigte Isocyanate.

6. Verwendung der gemäß Anspruch 1 bis 4 erhältlichen, olefinisch ungesättigten Isocyanate als Bindemittel für bei Raumtemperatur einkomponentig zu verarbeitende Beschichtungsmaterialien.

## Claims

1. Process for the preparation of olefinically unsaturated, urethane group-exhibiting isocyanates having an NCO content of from 4 to 20 wt-% and an (average) NCO functionality of from 0.6 to 1.5, characterised in that
a) an isocyanate component having an NCO content of from 20 to 56 wt-% and an (average) NCO functionality of less than 2.5, comprising substantially
a1) from 80 to 100 wt-% of urethane group-free cycloaliphatic diisocyanates having an NCO content of from 25 to 56 wt-% and
a2) from 0 to 20 wt-% of other organic polyisocyanates having an NCO content of from 10 to 50 wt-%
is reacted with
b) an olefinically unsaturated alcohol component having an (average) hydroxyl functionality of less than 1.5 and an iodine number greater than 70, comprising substantially
b1) from 80 to 100 wt-% of monohydric olefinically unsaturated alcohols or mixtures of such alcohols having (on average) 10 to 22 carbon atoms per molecule and
b2) from 0 to 20 wt-% of other mono- or polyhydric alcohols within the molecular weight range 32 to 326,
optionally in the presence of catalysts for the NCO/OH addition reaction, maintaining an NCO/OH equivalent ratio of from 4 to 40, and excess distillable starting isocyanates a1) and/or a2) are subsequently removed by distillation from the reaction products, down to a maximum residual content of such isocyanates of 0.5%.

2. Process according to Claim 1, characterised in that exclusively cycloaliphatic diisocyanates a1) are used as component a).

3. Process according to Claims 1 and 2, characterised in that exclusively 1-isocyanato-3,3,5-trimethyl-5-isocyanatomethyl cyclohexane is used as component a).

4. Process according to Claims 1 to 3, characterised in that exclusively fatty alcohols or mixtures of fatty alcohols having an iodine number greater than 90 and (on average) 14 to 20 carbon atoms per molecule are used as component b).

5. Olefinically unsaturated isocyanates obtainable according to Claims 1 to 4.

6. Use of the olefinically unsaturated isocyanates obtainable according to Claims 1 to 4 as binders for coating materials to be worked at room temperature as one-pot materials.

## Revendications

1. Procédé pour la préparation d'isocyanates à insaturation oléfinique contenant des groupes uréthannes, à une teneur en NCO de 4 à 20 % en poids et une fonctionnalité (moyenne) en groupes NCO de 0,6 à 1,5, caractérisé en ce que l'on fait réagir
a) un composant isocyanate à une teneur en NCO de 20 à 56 % en poids et une fonctionnalité (moyenne) en groupes NCO inférieure à 2,5, qui consiste essentiellement en
a1) 80 à 100 % en poids de diisocyanates cycloaliphatiques exempts de groupes uréthannes, à une teneur en NCO de 25 à 56 % en poids et
a2) 0 à 20 % en poids d'autres polyisocyanates organiques à une teneur en NCO de 10 à 50 % en poids,
avec
b) un composant alcool à insaturation oléfinique, ayant une fonctionnalité (moyenne) en groupes hydroxy inférieure à 1,5 et un indice d'iode supérieur à 70, qui consiste essentiellement en
b1) 80 à 100 % en poids d'alcools monovalents à insaturation oléfinique ou de mélanges de tels alcools contenant (en moyenne) 10 à 22 atomes de carbone par molécule et
b2) 0 à 20 % en poids d'autres alcools mono- ou poly-valents de poids moléculaire 32 à 326,
éventuellement en présence de catalyseurs de la réaction d'addition NCO/OH, en respectant un rapport de 4 à 40 entre les équivalents de groupes NCO et les équivalents de groupes OH, après quoi on débarrasse les produits de réaction, par distillation, de l'excès des isocyanates de départ a1) et/ou a2) qui sont distillables, jusqu'à une teneur résiduelle de 0,5 % au maximum en ces isocyanates.

2. Procédé selon la revendication 1, caractérisé en ce que l'on utilise en tant que composant a) exclusivement des diisocyanates cycloaliphatiques a1).

3. Procédé selon les revendications 1 et 2, caractérisé en ce que l'on utilise en tant que composant a) exclusivement le 1-isocyanato-3,3,5-triméthyl-5-isocyanatométhylcyclohexane.

4. Procédé selon les revendications 1 à 3, caractérisé en ce que l'on utilise en tant que composant b) exclusivement des alcools gras ou mélanges d'alcools gras ayant un indice d'iode supérieur à 90 et contenant (en moyenne) 14 à 20 atomes de carbone par molécule.

5. Isocyanates à insaturation oléfinique obtenus selon les revendications 1 à 4.

6. Utilisation des isocyanates à insaturation oléfinique obtenus selon les revendications 1 à 4 en tant que liants pour des produits de revêtement à utiliser en un seul composant à température ambiante.
